Europäisches Patentamt

European Patent Office

Office européen des brevets

(11) Publication number: **0 055 499**
A2

(12)

# EUROPEAN PATENT APPLICATION

(21) Application number: **81201348.0**

(22) Date of filing: **11.12.81**

(51) Int. Cl.³: **A 61 K 9/20**

(30) Priority: **22.12.80 US 219182**

(43) Date of publication of application:
**07.07.82 Bulletin 82/27**

(84) Designated Contracting States:
**BE CH DE FR GB IT LI LU NL**

(71) Applicant: **THE PROCTER & GAMBLE COMPANY**
**301 East Sixth Street**
**Cincinnati Ohio 45202(US)**

(72) Inventor: **Crosby, Thomas George**
**9355 Ranchill Drive**
**Cincinnati Ohio 45231(US)**

(74) Representative: **Suslic, Lydia et al,**
**Procter & Gamble European Technical Center**
**Temselaan 100**
**B-1820 Strombeek-Bever(BE)**

(54) Non-bleeding chewable tablets having high lipid content.

(57) A process for making non-bleeding, breakage resistant, chewable tablets, having a high content of lipid or lipid-like compounds, is disclosed. These tablets are particularly useful in formulating oral dosage forms of lipid-like vitamins or pharmaceutical agents. In the claimed process, the lipid, itself, or a freeze-dried powder made from a lipid emulsion is mixed with a specially selected, highly absorbent bulking/ binding agent to form a dry free-flowing powder. This powder is then compressed at relatively low pressure to form tablets. Tablets made by this process are also disclosed and claimed herein.

EP 0 055 499 A2

## NON-BLEEDING CHEWABLE TABLETS
## HAVING HIGH LIPID CONTENT

Thomas George CROSBY

### TECHNICAL FIELD

The present invention relates to a non-bleeding, breakage-resistant, chewable tablets which contain high levels of lipids or lipid-like compounds, and a process for making such tablets. The tablets are especially useful in providing a convenient route for the oral administration of vitamins and/or pharmaceutical agents having lipid-like characteristics.

### BACKGROUND OF THE INVENTION

Chewable tablets are a well-known method for providing food (e.g., chewable candies) and medicaments (e.g., vitamins or aspirin) to the consumer. The ability to chew and swallow the tablet is generally thought to be more convenient than tablets or capsules which have to be swallowed whole, particularly for children and older adults. However, when one of the basic ingredients to be incorporated in the tablet is a lipid or a material having lipid-like characteristics, it becomes extremely difficult to formulate tablets having high levels (i.e., greater than about 15%) of such materials.

The presence of high lipid levels tends to make the tablet unpalatable due to its unsavory taste and oily textural characteristics. Further, when such tablets are made in the conventional manner, i.e., by compression, the lipid material tends to "bleed" (leak) out of the tablet as the pressure is applied. This results in loss and waste of the lipid materials, as well as striation or layering in the tablet's internal structure, causing a highly friable and, therefore, fragile tablet. Such tablets are messy and costly to manufacture and difficult to handle without breaking. In addition, when the lipid material is a pharmaceutical agent, such bleeding makes dosage level control very difficult. It has now been found that by using the process of the present invention, these negative characteristics can be avoided, and nonbleeding, strong, chewable tablets, having a high content of lipids or lipid-like compounds, can be formed in a simple and efficient manner.

The use of whey and other modified milk products in the manufacture of powdered food products, such as milk drinks, shortenings, and salad dressings, which are stored as powders and are reconstituted by the addition of a liquid, such as water or milk, is well-known. Generally, in such products, a food component or components, such as an edible oil, is added to whey together with various other food components, additives or stablilizers, and the resulting mixture is spray-dried to form a dry, free-flowing powder. See, for example, German Offenlegungs-schrift 2,027,679, Bratland, published December 17, 1970; U.S. Patent 3,560,220, Bangert, et al., issued February 2, 1971; British Patent Specification 1,368,229, Chemische Fabrik Stockhausen, published September 25, 1974; and U.S. Patent 1,302,486, Dunham, issued April 29, 1919, all of which are incorporated herein by reference. The powdered products made by such a process are the end result sought in this art and little, if any, thought is given in the art to the problems which are encountered when conventional tableting operations are applied to materials having a high lipid content.

Spray-dried or freeze-dried whey is known as a carrier material for liquid substances and as a bulking agent for encapsulated pharmaceutical products. See, for example, U.S. Patent 3,582,362, Drews, et al., issued June 1, 1971; U.S. Patent 3,617,302, Collins, issued November 2, 1971; and Kraflow Trade Bulletin, issued by Kraftco Corp., all of which are incorporated herein by reference. Again, these materials relate only to powdered compositions, without any consideration given to the effect of compression in a tableting operation on materials having a high lipid content, which is the situation where the primary benefits of the present invention are seen.

It is, therefore, an object of the present invention to provide a simple and efficient process for producing chewable tablets having a high content of lipids or lipid-like compounds.

It is a further object of the present invention to provide a process which produces non-bleeding, breakage resistant, chewable tablets containing at least about 15% of lipid or lipid-like compounds.

It is a still further object of the present invention to provide chewable tablets, having high lipid content, which are palatable, non-bleeding and breakage resistant.

The present invention relates to a process for making a non-bleeding, chewable tablet containing at least about 15% of an edible lipid or lipid-like compound, comprising the steps of:

> (a)  combining a lipid material with an edible binding/bulking agent capable of completely absorbing the quantity of lipid material so included, to form a dry, free-flowing powder; and
>
> (b)  compressing said powder at a pressure from about 25 to about 5,000 pounds per square inch to form the tablet.

In a preferred process, the lipid-like compound included in the tablets is a vitamin or pharmaceutical agent, particularly a polyol fatty acid ester having at least 4 fatty acid ester groups, each fatty acid having from about 8 to about 22 carbon atoms. A preferred binding/ bulking agent for use in the present invention is spray-dried or freeze-dried whey. The chewable tablets made by the process of the present invention are also disclosed and claimed.

## DETAILED DESCRIPTION OF THE INVENTION

The process of the present invention utilizes a lipid or lipid-like compound, or mixtures of such compounds, and incorporates them into chewable tablet form. These lipid or lipid-like compounds may be food ingredients, such as oils (e.g., vegetable oil, soybean oil, corn oil, sunflower oil, safflower oil, or rapeseed oil), fats (e.g., animal fat or butter fat), hardened fats or oils, flavorants (e.g., orange oil, lemon oil, or peppermint oil), or they may be vitamins or pharmaceutical agents having lipid-like characteristics.

When pharmaceutical agents are used, they are incorporated in amounts which are both safe and effective in the treatment of a particular medical condition; the determination of what constitutes "a safe and effective amount" will be dependent upon the particular pharmaceutical agent to be incorporated and is well within the skill of those knowledgeable in the pharmaceutical arts. In the pharmaceutical category, polyol fatty acid esters having at least 4 fatty acid ester groups, each fatty acid having from about 8 to about 22 carbon atoms, are preferred. These compounds are known to be useful as low calorie fats and as agents for inhibiting the absorption of cholesterol in the body; they are are described in detail in U.S. Patent 3,600,186, Mattson and Volpenhein, issued August 17, 1971; and U.S. Patent 3,954,976, Mattson and Volpenhein, issued May 4, 1976, both of which are incorporated herein by reference.

Preferred polyol polyester compounds for use herein include sucrose hexa- through octaoleates, particularly sucrose octaoleate, while other useful polyol polyesters include glucose tetraoleate, glucose tetrastearate, glucose tetraester of soybean oil fatty acid, mannose tetraester of tallow fatty acid, galactose tetraester of olive oil fatty acid, arabinose tetraester of cottonseed oil fatty acid, xylose tetralinoleate, galactose pentastearate, sorbitol tetraoleate, sorbitol hexa-ester of olive oil fatty acid, xylitol pentapalmitate, xylitol tetraester of substantially completely hydrogenated cottonseed oil fatty acid, sucrose tetrastearate, sucrose pentastearate, sucrose hexaoleate, sucrose octaester of substantially completely hydrogenated soybean oil fatty acid, sucrose octaester of peanut oil fatty acid, erythritol tetraester of olive oil fatty acid, erythritol tetraoleate, xylitol pentaoleate, sorbitol hexaoleate, sucrose octaester of soybean oil fatty acid, and mixtures of these compounds.

The lipids or lipid-like compounds are incorporated in tablets of the present invention at a level of at least 15%, and preferably (especially in the case of the polyol polyesters, described above) at least about 30% of the finished tablet composition. The lipid or lipid-like compounds can be incorporated into the tablet in either of two forms: (1) as the compound, itself or (2) as a freeze-dried powder made from a water-stable emulsion containing at least 50% of the lipid or lipid-like compound. Although these options represent two different starting materials for the process of the present invention, for the sake of convenience they are both referred to generically as "lipid material" in the present application.

In forming the water-stable emulsions, described above, the emulsifier systems described in U.S. Patent 3,949,102, Hellyer, et al., issued April 6, 1976 and U.S. Patent 3,968,266, Baugher, issued July 6, 1976, both of which are incorporated herein by reference, are highly preferred. These emulsifier systems contain from about 0.5 parts to about 18 parts of a first emulsifier selected from the group consisting of edible polyglycerol fatty acid esters and edible sorbitan fatty acid esters, together with from about 0.5% to about 15% by weight of said first emulsifier of a second emulsifier which is an anionic surfactant. Polyglycerol esters suitable for use in this emulsifier system have an average of from about 2 to 10 glycerol units and from 1 to 3 fatty acyl groups of from 14 to 18 carbon atoms per glycerol moiety. Preferred polyglycerol esters have an average of 2 or 3 glycerol units and 1 fatty acyl group having from 14 to 18 carbon atoms per glycerol moiety. Sorbitan esters suitable for use in the emulsifier system have from 1 to 3 fatty acyl groups having from about 14 to about 18 carbon atoms each. Preferred sorbitan esters have 1 fatty acyl group having 14 to 18 carbon atoms. Examples of anionic emulsifiers suitable for use in these emulsifier systems include the alkali and alkaline earth metal salts of fatty carboxylates, sulfates, and sulfonates, half salts of dicarboxylic fatty acid esters and salts of fatty acid lactylates having from 8 to about 20 carbon atoms in the anionic moiety.

The binding/bulking agents useful in the process of the present invention must be edible and capable of completely absorbing the high levels of lipids or lipid-like compounds utilized, forming a dry, free-flowing powder. The absorption capacity of the binding/bulking agent must be sufficient to permit incorporation of the necessary amount of lipid or lipid-like material in a tablet of reasonable size. Examples of useful binding/ bulking agents include polyvinyl pyrrolidone, methyl cellulose, dried, freeze-dried or spray-dried whey, freeze-dried or spray-dried maltodextrin, and mixtures of these materials. Freeze-dried or spray-dried starch, such as that described in U.S. Patent 3,769,038, incorporated herein by reference, may also be used as the binding/bulking agent. A particularly preferred binding/bulking agent is freeze-dried or spray-dried whey, such as that sold under the tradename Kraflow by Kraftco Corp., New York, New York. These materials are described in detail in U.S. Patent 3,582,362, Drews, et al., issued June 1, 1971, and U.S. Patent 3,617,302, Collins, issued November 2, 1971, both of which are incorporated herein by reference. The preferred binding/bulking agents can be made by a gas injection spray drying technique and have a substantially spherical, expanded structure; they generally have bulk densities in the range of from about 0.3 grams per cubic centimeter to about 0.03 grams per cubic centimeter and a particle size of from about 0.001 inch to about 0.1 inch (e.g., 95% of the particles pass through a Standard U.S. #40 screen-sieve opening = 420 microns). Another example of a spray-dried whey is Amberpro, commercially available from Amber Laboratories, Juneau, Wisconsin. A commercially available spray-dried maltodextrin is Maltrin, sold by Grain Processing Corp., Muscatine, Iowa.

A suitable binding/bulking agent for use in the present invention can be made in the following manner. Sweet whey, obtained from a cottage cheese made process, is concentrated to a level of 35% solids. The whey is pumped at a pressure of 2200 psi to a single fluid nozzle in a spray-drying chamber. Prior to reaching the nozzle, air is injected into the flowing stream of whey at a level of 2500 psi. The whey is then atomized in the spray-drying chamber and is dried to a level of 3.5% moisture. The whey has a bulk density of 0.08 grams per cubic centimeter.

In preferred embodiments of the present invention, from about 10% to about 60% of the binding/bulking agent (especially freeze-dried or spray-dried whey) is replaced by a 2-stage dried whey material, such as Krafen, available from Kraftco Corp., New York, New York. The 2-stage dried whey acts to densify the powder formed, and provides a final tablet which disperses easily in the mouth and which does not possess a sticky or gummy mouthfeel.

In the process of the present invention, the lipid material (in the form of the lipid, itself, or as a freeze-dried lipid emulsion) and binding/bulking agent are mixed together, at a weight ratio of lipid material:binding/ bulking agent of from about 1:5 to about 5:1, preferably from about 1:3 to about 3:1, to form a dry, free-flowing powder.

Where the lipid compound, itself, rather than the freeze-dried lipid emulsion, is used, conventional processes are used to apply the lipid to the binding/bulking agent. It is desirable to use dry mixing equipment, such as kitchen-type mixers or ribbon blenders, that is not abusive to the binding/bulking agent. For certain types of binding/bulking agents, such as the spray-dried or freeze-dried whey, the level of breakdown may be reduced by the inclusion of strengthening agents, such as film-forming protein. Such proteins as sodium caseinate, egg albumin or gelatin may be used. The protein acts to provide a stronger particle, which is less subject to breakdown during mixing.

The dry, free-flowing powder is then made into tablets using conventional tableting equipment and presses, exerting a relatively low tableting pressure. The compression used is in the range of from about 25 to about 5,000, preferably from about 100 to about 5,000, more preferably from about 500 to about 1,500, psi. Lower pressures (e.g., from about 25 to about 500 psi) are especially useful in the manufacture of small (<1 inch diameter) tablets. The resulting tablets are attractive aesthetically, palatable, and are non-bleeding and breakage resistant.

The tablets of the present invention may also optionally contain other components conventionally found in food or pharmaceutical compositions, in their art-established levels of use. Examples of such components include binders, and bulking agents not falling within the above definitions, vitamins, minerals, glidants, dissolution agents, emulsifiers, preservatives, anti-oxidants, starches, flour, milk or milk extracts, such as milk sugar or sodium caseinate, sweeteners

0055499

- 9 -

and flavorants, colorants, vegetable proteins, gelatin, vegetable gums, food acids, and mixtures of the foregoing. In addition, the tablets may be coated, such as by a sugar or enteric coating.

Unless otherwise specified all percentages and ratios given herein are by weight.

The following non-limiting examples illustrate the processes and tablets of the present invention.

### EXAMPLE I

A total of 3 grams of vegetable oil (Crisco[R] Oil, sold by The Procter & Gamble Company, Cincinnati, Ohio) was added to 7 grams of spray-dried whey (Kraflow, sold by Kraftco, Corp., New York, New York) in a beaker, by first taking a portion of the Kraflow and making a paste with the oil. Then the remaining portions of the Kraflow material were added to the paste and blended until it was all homogeneously added; a dry, flowable powder resulted. A tablet, 1.5 inches in diameter, was manually pressed from this dry powder mixture utilizing a Carver tableting press, Model C. The compression pressure was 1000 psig.

The resulting tablet was chewable, exhibited no surface oil bleeding, had a homogeneous structure and was not easily breakable.

Substantially similar results are achieved where the vegetable oil used in the above procedure is replaced, in whole or in part, with a polyol fatty acid ester having at least 4 fatty acid ester groups, each fatty acid group having from about 8 to about 22 carbon atoms, such as sucrose octaoleate. Similar results are also achieved where the Kraflow binding/bulking agent is replaced, in whole or in part, by freeze-dried or spray-dried maltodextrin, freeze-dried or spray-dried starch, polyvinyl pyrrolidone or methyl cellulose. Similar results are obtained where the Crisco oil is formed into a water-stable emulsion by combining it with triglycerol monostearate and sodium oleate; this emulsion being freeze-dried to form a dry powder which is then mixed with the Kraflow, and formed

- 10 -

into a tablet as described above. Substantially similar results are also obtained when the Crisco Oil is replaced, in whole or in part, by cottonseed oil, corn oil, safflower oil, rapeseed oil, palm kernel oil, coconut oil, sunflower oil, or mixtures of these materials.

### EXAMPLE II

Non-bleeding, breakage resistant chewable tablets were processed using the four formulations given below.

| Component | Weight (grams) | | | |
|---|---|---|---|---|
| | 1 | 2 | 3 | 4 |
| Vegetable Oil (Crisco) | 3.00 | 3.00 | 3.00 | 3.00 |
| Whey (Kraflow) | 6.50 | 5.00 | 4.00 | 5.00 |
| Powdered Malt | 0.25 | 0.25 | 0.25 | — |
| Powdered Vanilla | 0.25 | 0.25 | 0.25 | 0.50 |
| Maltrin 150 | — | — | 2.50 | — |
| Mannitol | — | 1.50 | — | 1.50 |

The dry tablet ingredients were first blended until homogeneous; then the oil was added until a uniform dry powder was formed. The tablets, 1 1/2 inches in diameter, were made by compression of the powder at about 1,000 psi.

### EXAMPLE III

A larger batch of tablet mixture was prepared in a 100 gallon stainless steel ribbon blender. The formulation is given in the table below.

| Component | Weight (lbs.) |
|---|---|
| Whey (Kraflow) | 40.4 |
| Vegetable Oil (Crisco) | 19.9 |
| Mannitol | 3.4 |
| Powdered Malt | 1.7 |
| Powdered Vanilla | 0.8 |

- 11 -

The dry ingredients were charged and mixed thoroughly in the blender. About half the oil was sprayed into the mixed powder, with agitation, using a commercial paint sprayer. The remainder of the oil was poured slowly into the mixture with continued agitation. Periodically the agitation was stopped and the mixer surface was scraped. The contents were mixed for two hours and the result was a dry, free-flowing powder. The powder bulk density was increased by manually compressing the powder with the bottom of a stainless steel container and then breaking up the resulting loose cake with a spatula. Tablets (1 1/2 inch diameter), weighing 8.5 grams, were compressed from this powder at pressures ranging from 500 to 2,000 psi. The tablets were uniform and exhibited no surface bleeding, except at the highest tableting pressure where a small amount of surface bleeding was noted.

## EXAMPLE VI

A tablet of improved texture and mouthfeel was formulated as follows.

| Component | Weight (grams) |
| --- | --- |
| Vegetable Oil | 2.55 |
| Spray-dried whey (Kraflow) | 4.00 |
| 2-Stage Dried Whey (Krafen) | 4.00 |
| Powdered Malt | 0.09 |
| Powdered Vanilla | 0.25 |
| Mannitol | 0.51 |

The dry ingredients were first blended until homogeneous, then the oil was added, with mixing, until a uniform dry powder was formed. A non-bleeding, chewable tablet, 1 1/2 inches in diameter, was formed by compressing the powder at about 500 psi. This tablet dissolved more readily in the mouth than one in which the 2-stage dried whey is removed and replaced by an equivalent amount of spray-dried whey.

- 12 -

Substantially similar results are obtained where the vegetable oil used in the above procedure is replaced, in whole or in part, with a polyol fatty acid ester having at least four fatty acid ester groups, each fatty acid group having from about 8 to about 22 carbon atoms, such as sucrose octaoleate.

- 1 -

CLAIMS

1. A process for making a non-bleeding, chewable tablet containing at least 15% of an edible lipid or lipid-like compound, characterized in that it comprises the steps of:

   (a) combining a lipid material with an edible binding/bulking agent capable of completely absorbing the quantity of lipid material so included, to form a dry, free-flowing powder; and

   (b) compressing said powder at a pressure of from 25 to 5,000 psi to form the tablet.

2. A process according to Claim 1 characterized in that said lipid material is a freeze-dried powder formed from a water-stable emulsion containing at least 50% of the edible lipid or lipid-like compound.

3. A process according to Claim 1 characterized in that the lipid material is the lipid or lipid-like compound, itself.

4. A process according to any one of Claims 1-3 characterized in that the binding/bulking agent is selected from the group consisting of polyvinyl pyrrolidone, methyl cellulose, freeze-dried or spray-dried starch, freeze-dried or spray-dried maltodextrin, dried, freeze-dried or spray-dried whey, or mixtures thereof.

5. A process according to any one of Claims 1-4 characterized in that said binding/bulking agent is freeze-dried or spray-dried whey having a substantially spherical, expanded structure, a bulk density of from 0.05 to 0.3 grams per cubic centimeter and a particle size of from 0.001 to 0.1 inch.

- 2 -

6. A process according to any one of Claims 1-5 characterized in that the lipid compound is a polyol fatty acid ester having at least 4 fatty acid ester groups, each fatty acid group having from 8 to 22 carbon atoms.

7. A process according to any one of Claims 1-6 characterized in that said lipid component is sucrose octaoleate.

8. A process according to any one of Claims 1-7 characterized in that the ratio, by weight, of the lipid material to the binding/bulking agent is from 1:5 to 5:1.

9. A process according to any one of Claims 1-8 characterized in that the tableting pressure is from 500 to 1,500 psi.

10. A non-bleeding, chewable tablet characterized in that it is made by the process according to any one of Claims 1-9.